Europäisches Patentamt

European Patent Office

Office européen des brevets

(19)

(11) Veröffentlichungsnummer: **0 024 046**
**B1**

(12) # EUROPÄISCHE PATENTSCHRIFT

(45) Veröffentlichungstag der Patentschrift:
**14.12.83**

(21) Anmeldenummer: **80104695.4**

(22) Anmeldetag: **09.08.80**

(51) Int. Cl.³: **A 61 M 5/20,** A 61 M 5/32

(54) **Injektionsspritze.**

(30) Priorität: **13.08.79 DE 2932719**

(43) Veröffentlichungstag der Anmeldung:
**18.02.81 Patentblatt 81/7**

(45) Bekanntmachung des Hinweises auf die Patenterteilung:
**14.12.83 Patentblatt 83/50**

(84) Benannte Vertragsstaaten:
**AT BE CH FR IT LI NL SE**

(56) Entgegenhaltungen:
**US - A - 3 556 100**
**US - A - 4 194 505**

(73) Patentinhaber: **Lucas, Dieter, Dr., Schlosstrasse 5,
D-7763 Öhningen-Kattenhorn (DE)**

(72) Erfinder: **Lucas, Dieter, Dr., Schlosstrasse 5,
D-7763 Öhningen-Kattenhorn (DE)**

(74) Vertreter: **Hiebsch, Gerhard F., Dipl.-Ing.,
Erzbergerstrasse 5A Postfach 464, D-7700 Singen 1 (DE)**

Injektionsspritze

Die Erfindung betrifft eine Injektionsspritze mit in einem Gehäuse vorgesehenem Aufnahmeraum für eine unter Druck eines Kolbens setzbare Injektionsflüssigkeit sowie mit einer durch einen Kolben bewegbaren Injektionsnadel.

Es sind Wegwerfspritzen bekannt, bei denen die Nadel auf die Spritze aufgeschraubt werden muss, um sie funktionsfähig zu machen. Die Injektionsflüssigkeit wird aus Ampullen entnommen und in die Spritze aufgezogen. Die Manipulation mit derartigen Spritzen ist hygienisch nicht einwandfrei und darüber hinaus zeitraubend.

Weiterhin zeigt die US-A-3 556 100 eine Spritze, bei welcher sich die Injektionsnadel und die Injektionsflüssigkeit in einem Raum befinden. Unter dem Druck einer Schraubenfeder wird die Nadel aus dem Raum gestossen und gleichzeitig die Flüssigkeit durch eine Nadelkanüle gepresst.

Diese Anordnung hat zum einen den Nachteil, dass Nadel und Flüssigkeit in ständigem Kontakt miteinander stehen, wobei Korrosionserscheinungen an der Nadel wegen der oftmals aggressiven Flüssigkeiten oder Verunreinigungen der Flüssigkeit nicht ausgeschlossen sind. Weiterhin wird in jedem Fall mit der Nadel auch die Flüssigkeit freigesetzt, so dass es nicht möglich ist, zuerst die Nadel beispielsweise in das Muskelgewebe eines Patienten einzubringen und dann die Zugabe der Flüssigkeit vorzunehmen.

Angesichts dieser Gegebenheiten hat sich der Erfinder das Ziel gesetzt, eine Injektionsspritze der eingangs erwähnten Art zu schaffen, welche diese Nachteile vermeidet und insbesondere eine flexiblere Handhabung gewährleistet.

Zur Lösung dieser Aufgabe führt, dass die Injektionsnadel mit einem sich entgegen der Kolbenschubrichtung konisch erweiternden Innenraum versehen ist, dass die Injektionsnadel in einem dem Aufnahmeraum für die Injektionsflüssigkeit anliegenden, jedoch von diesem getrennten Nadelraum untergebracht und von dem Kolben aus dem Nadelraum ausschiebbar ist und dass der Innenraum mit dem Aufnahmeraum in einer Betriebsstellung verbindbar ist.

Die Injektionsnadel ist mit einem sich gegen die Kolbenschubrichtung konisch erweiternden Innenraum versehen. Sie ist mit dem Kolben aus dem Nadelraum in eine Betriebsstellung überführbar gestaltet. Hierzu lagert die Nadel der erfindungsgemässen Injektionsspritze bevorzugtermassen in einem Zylinderraum, in welchem die am Kolben befestigte Nadel durch den Druck eines Kraftspeichers verschoben werden kann und dabei aus dem Zylinder austritt, um die sogenannte Betriebsstellung einzunehmen. Die Nadel tritt also erst aus ihrer Umhüllung hervor, wenn sie verwendet wird und kann vorher nicht verschmutzen.

Auch der Kolben zum Ausschieben der Flüssigkeit steht unter dem Druck eines Kraftspeichers. Beide sind in Ruhelage der erfindungsgemässen Spritze gespannt und werden von Verriegelungs- oder Verschlusselementen gehalten, welche einfach bedienbar sein müssen. Hierbei hat sich eine Hebeleinrichtung als äusserst wirksam erwiesen, welche einerseits ein Rastorgan des Nadelkolbens bzw. des Druckbolzens hält und anderseits den Kolben für die Flüssigkeit, wobei – in einem anderen Merkmal der Erfindung – der Flüssigkeitskolben von der Hebeleinrichtung in zeitlicher Verzögerung zur Freisetzung des Nadelkolbens freigebbar ausgebildet ist. Dank dieser Massgabe rastet zuerst die Injektionsnadel in ihrer sogenannten Betriebsstellung ein, bevor sie mit Flüssigkeit beaufschlagt wird, was das Einsatzgebiet der erfindungsgemässen Injektionsspritze erheblich erweitert und insbesondere keine Probleme mit der sogenannten Aspiration aufkommen lässt.

Des weiteren hat es sich als günstig erwiesen, den Zylinderboden des Aufnahmeraumes für die Injektionsnadel aus einem von der Nadelspitze perforierbaren Werkstoff herzustellen, der bevorzugt auch die Eigenschaft aufweisen soll, die Nadel nach der Perforation manschettenartig zu umgeben, und zwar etwa in der Art eines sich an die Nadel anliegenden Gummis.

Die beiden benachbarten Aufnahmeräume für Nadel und Flüssigkeit sind erfindungsgemäss mittels eines Durchbruches im Bereich der Zylinderböden verbunden und in Ruhelage der Injektionsspritze durch ein Verschlusselement, beispielsweise ein Siegelplättchen, voneinander zeitweilig getrennt. Das Siegelplättchen ist so gestaltet, dass es beim Auftreffen des Nadelkolbens entfernt oder zerschlagen wird und so den Durchlass für die Flüssigkeit freigibt, welche dann in den Hohlraum der Nadel eindringen kann.

Weitere Vorteile, Merkmale und Einzelheiten der Erfindung ergeben sich aus der nachfolgenden Beschreibung eines bevorzugten Ausführungsbeispiels sowie anhand der Zeichnung; diese zeigt in:

Fig. 1: den Längsschnitt durch ein Ausführungsbeispiel in Ruhelage;

Fig. 2: die Injektionsspritze nach Fig. 1 in Betriebsstellung;

Fig. 3: die Injektionsspritze nach Fig. 1, 2 in entleertem Zustand.

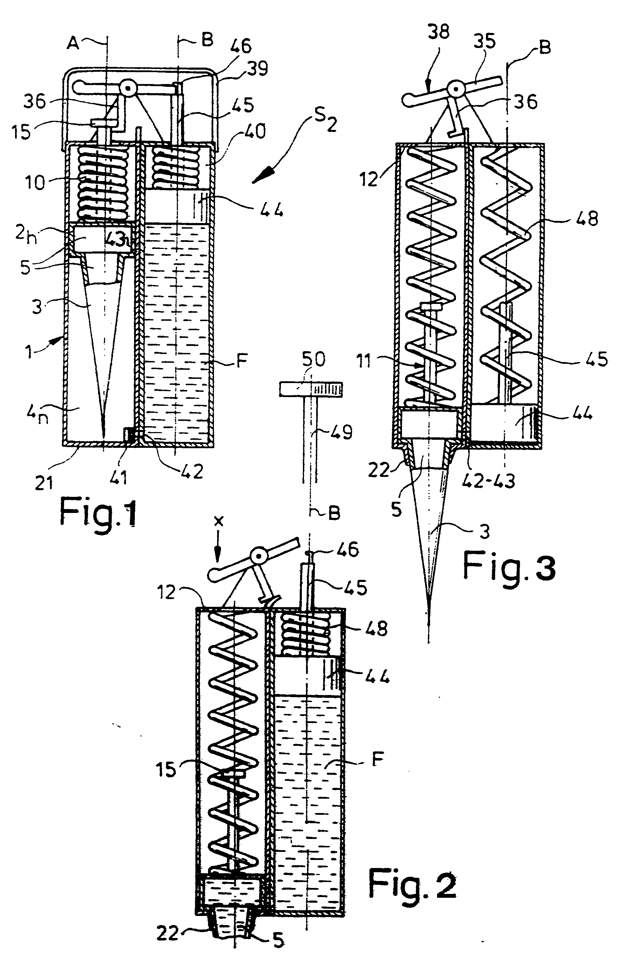

Eine Injektionsspritze $S_2$ für flüssige Medikamente besteht nach Fig. 1 aus zwei Aufnahmeräumen 40, mit parallelen Längsachsen A, B. Der eine Aufnahmeraum $4_n$ enthält eine Hohlnadel 3, während eine Injektionsflüssigkeit F im parallelen Aufnahmeraum 40 untergebracht ist.

Die Hohlnadel 3 ist in einem Zylinder 1 angeordnet und an ihrem nadelspitzenfernen Ende mit einem hohlen Kolben $2_h$ verbunden, auf welchen in der Spritzenlängsachse A ein durch eine Feder 10 belasteter Hubbolzen 11 drückt. Diese Schraubenfeder 10 stützt sich einerseits gegen den Kolben $2_h$ und anderseits gegen die Innenseite einer Widerlagerplatte 12 ab.

Nach oben hin endet der Druckbolzen 11 in einem Bolzenkopf 15. Nach dem Abnehmen einer Schutzkappe 39 wird durch Druck (Pfeil x) auf einen Hebelverschluss 38 der Bolzenkopf 15 freigegeben und der hohle Kolben $2_h$ von der Feder 10 abwärts zum Zylinderboden 21 geführt, wobei eine seitliche Versiegelung 41 zerstört wird; diese verschliesst einen Durchgang 42 zum benachbarten Aufnahmeraum 40, welcher mittels Perforationen 43 des Hohlkolbens $2_h$ mit dem Nadelhohlraum 5 verbunden wird. In diesen strömt sofort unter Druck eines von einer Feder 48 belasteten Kolbens 44 die Flüssigkeit F ein (siehe Fig. 2 und 3). Beim Abwärtsführen des Kolbens $2_h$ zum Zylinderboden 21 durchstösst die Nadel 3 diesen, wobei sich dessen Reste als Manschette 22 der Hohlnadel 3 seitlich anschmiegen.

Ein axialer Führungsstab 45 des Flüssigkeitskolbens 44 hält in Ruhelage nach Fig. 1 einen Haken 36 durch Druck eines Mitnehmers 46 gegen einen Kipphebel 35 unter dem Bolzenkopf 15 – und diesen damit in Verriegelungs- oder Verschlussstellung. Mit Zeitverzögerung gegen die Absenkung des Druckbolzens 11 löst sich jener Kipphebel 35 vom Führungsstab 45, so dass dieser den Flüssigkeitskolben 44 freisetzt. Die Zeitverzögerung kann beispielsweise durch nicht näher dargestellte Kulissenelemente erzeugt werden. Um ein langsames Verschieben des Flüssigkeitskolbens 44 zu ermöglichen, kann dessen Schaft 45 noch mit einem Handgriff 50 verbunden werden, welcher seinerseits einen Schaft 49 aufweist.

**Patentansprüche**

1. Injektionsspritze ($S_2$) mit in einem Gehäuse vorgesehenem Aufnahmeraum (40) für eine unter Druck eines Kolbens (44) setzbare Injektionsflüssigkeit (F) sowie mit einer durch einen Kolben ($2_h$) bewegbaren Injektionsnadel (3), dadurch gekennzeichnet, dass die Injektionsnadel (3) mit einem sich entgegen der Kolbenschubrichtung konisch erweiternden Innenraum (5) versehen ist, dass die Injektionsnadel (3) in einem dem Aufnahmeraum (40) für die Injektionsflüssigkeit (F) anliegenden, jedoch von diesem getrennten Nadelraum ($4_n$) untergebracht und von dem Kolben ($2_h$) aus dem Nadelraum ($4_n$) ausschiebbar ist und dass der Innenraum (5) mit dem Aufnahmeraum (40) in einer Betriebsstellung verbindbar ist.

2. Injektionsspritze nach Anspruch 1, dadurch gekennzeichnet, dass der Kolben ($2_h$) für die Injektionsnadel (3) und der Kolben (44) für die Flüssigkeit (F) in Ruhelage der Injektionsspritze ($S_2$) unter Druck jeweils eines Kraftspeichers (10, 48) stehen, der in seiner Ruhe- oder Spannlage von einem Verschlusselement (38) gehalten ist.

3. Injektionsspritze nach Anspruch 2, dadurch gekennzeichnet, dass als Verschlusselement eine Hebeleinrichtung (38) dient, welche einerseits ein Rastorgan (15) des Nadelkolbens ($2_h$) bzw. Druckbolzens (11) hält und anderseits den Kolben (44) für die Flüssigkeit.

4. Injektionsspritze nach Anspruch 3, dadurch gekennzeichnet, dass der Flüssigkeitskolben (44) von der Hebeleinrichtung (38) in zeitlicher Verzögerung zur Freisetzung des Nadelkolbens ($2_h$) freigebbar ausgebildet ist.

5. Injektionsspritze nach wenigstens einem der Ansprüche 1 bis 4, dadurch gekennzeichnet, dass der Zylinderboden (21) des Aufnahmeraumes ($4_n$) für die Injektionsnadel (3) aus einem von der Nadel (3) perforierbaren Werkstoff besteht, der die Injektionsnadel (3) nach der Perforierung manschettenartig (22) umgibt.

6. Injektionsspritze nach wenigstens einem der Ansprüche 1 bis 5, dadurch gekennzeichnet, dass der Aufnahmeraum ($4_n$) für die Nadel (3) mit dem Flüssigkeitsraum (40) mittels eines Durchbruches (42) in der Nähe des Zylinderbodens (21) verbunden und in Ruhelage der Spritze ($S_2$) der Durchbruch verschlossen ist.

7. Injektionsspritze nach Anspruch 6, dadurch gekennzeichnet, dass der Verschluss (41) des Durchbruches (42) durch die Injektionsnadel (3) oder den Kolben ($2_h$) zerstörbar oder beseitigbar angeordnet ist.

8. Injektionsspritze nach Anspruch 6 oder 7, dadurch gekennzeichnet, dass der Durchbruch (42) zwischen den benachbarten Aufnahmeräumen ($4_n$, 40) in Betriebsstellung der Injektionsspritze ($S_2$) mit einer Perforation (43) des Nadelkolbens ($2_h$) fluchtet.

**Claims**

1. Injection syringe ($S_2$) with a receiving chamber (40) provided in a housing, for an injection fluid (F) which can be put under the pressure of a piston (44), and also with an injection needle (3), movable by a piston ($2_h$), characterised in that, the injection needle (3) is provided with an internal chamber (5) widening conically opposite the direction of the piston stroke, that the injection needle (3) is accommodated in a needle chamber ($4_n$), which is adjacent to the receiving chamber (40) for the injection fluid (F), but separated from it, and is movable by the piston ($2_h$) out of the needle chamber ($4_n$), and that the internal chamber (5) can be connected with the receiving chamber (40) in an operating position.

2. Injection syringe according to claim 1, characterised in that the piston ($2_h$) for the injection needle (3) and the piston (44) for the fluid (F), in the rest condition of the injection syringe ($S_2$), are each under the pressure of an energy storage device (10, 48), which in its rest or stressed condition is retained by a locking element (38).

3. Injection syringe according to claim 2, characterised in that a lever device (38) acts as locking element, which on the one hand retains a latching member (15) of the needle piston ($2_h$) or as the case may be a pressure pin (11), and on the other hand retains the piston (44) for the fluid.

4. Injection syringe according to claim 3, characterised in that the fluid piston (44) is formed to be releasable from the lever device (38), with delay in time in relation to the release of the needle piston ($2_h$).

5. Injection syringe according to at least one of claims 1 to 4, characterised in that the cylinder floor (21) of the receiving chamber (4$_n$) for the injection needle (3) consists of a material which can be perforated by the needle (3), which, after the perforating, surrounds the injection needle (3) in the manner of a collar (22).

6. Injection syringe according to at least one of claims 1 to 5, characterised in that the receiving chamber (4$_n$) for the needle (3) is connected with the fluid chamber (40) by means of an aperture (42) in the neighbourhood of the cylinder floor (21), and in the rest condition of the syringe (S$_2$) the aperture is closed.

7. Injection syringe according to claim 6, characterised in that the closure member (41) for the aperture (42) is arranged to be disturbed or displaced by the injection needle (3) or the piston (2$_h$).

8. Injection syringe according to claim 6 or 7, characterised in that the aperture (42) between the adjoining receiving chambers (4$_n$, 40) is in alignment with a perforation (43) of the needle piston (2$_h$), in the operation position of the injection syringe (S$_2$).

**Revendications**

1. Seringue à injection (S$_2$), présentant une chambre de réception (40) prévue dans un corps pour un liquide à injecter (F) qui peut être mis sous la pression d'un piston (44), ainsi qu'une aiguille à injection (3) qui peut être déplacée par un piston (2$_h$), caractérisée en ce que l'aiguille à injection (3) est munie d'une chambre intérieure (5) qui s'élargit en cône en sens inverse du sens de la poussée du piston, que l'aiguille à injection (3) est logée dans une chambre d'aiguille (4$_n$), adjacente à la chambre (40) de réception du liquide à injecter (F) mais séparée de celle-ci et peut être éjectée de la chambre d'aiguille (4$_n$) par le piston (2$_h$) et en ce que la chambre intérieure (5) peut être reliée à la chambre de réception (40) dans une position de service.

2. Seringue à injection selon la revendication 1, caractérisée en ce que, dans la position de repos de la seringue à injection (S$_2$), le piston (2$_h$) prévu pour l'aiguille d'injection (3) et le piston (44) prévu pour le liquide (F) sont soumis chacun à la pression d'un accumulateur de force (10, 48) qui est maintenu dans sa position de repos ou position armée par un élément d'enclenchement (38).

3. Seringue à injection selon la revendication 2, caractérisée en ce qu'elle comporte, comme élément d'enclenchement, un dispositif à levier (38) qui retient, d'une part, un organe d'arrêt (15) du piston (2$_h$) de l'aiguille ou de la tige de pression (11) et, d'autre part, le piston (44) prévu pour le liquide.

4. Seringue à injection selon la revendication 3, caractérisée en ce que le piston à liquide (44) est agencé pour pouvoir être libéré par le dispositif à levier (38) avec retardement dans le temps par rapport à la libération du piston (2$_h$) de l'aiguille.

5. Seringue à injection selon l'une quelconque des revendications 1 à 4, caractérisée en ce que le fond (21) du cylindre de la chambre (4$_n$) de logement de l'aiguille d'injection (3) est constitué par une matière perforable par l'aiguille (3) et qui entoure l'aiguille d'injection (3) à la façon d'une manchette (22) après la perforation.

6. Seringue à injection selon l'une quelconque des revendications 1 à 5, caractérisée en ce que la chambre (4$_n$) de réception de l'aiguille (3) est reliée à la chambre à liquide (40) par une ouverture (42) ménagée dans le voisinage du fond (21) du cylindre et en ce que cette ouverture est obturée dans la position de repos de la seringue (S$_2$).

7. Seringue à injection selon la revendication 6, caractérisée en ce que l'obturateur (41) de l'ouverture (42) est agencé de manière à pouvoir être détruit ou écarté par l'aiguille à injection (3) ou par le piston (2$_h$).

8. Seringue à injection selon la revendication 6 ou 7, caractérisée en ce que l'ouverture (42) entre les chambres de réception voisines (4$_n$, 40) est alignée sur une perforation (43) du piston (2$_h$) de l'aiguille dans la position de service de la seringue à injection (S$_2$).

Fig.1

Fig.2

Fig.3